# EUROPEAN PATENT APPLICATION

(11) **EP 3 838 140 A1**
(43) Date of publication of application: **23.06.2021**
(21) Application number: 19218617.9
(22) Date of filing: 20.12.2019
(51) Int. Cl.: A61B 5/107, A61B 5/00, G06T 7/50, A61B 5/11, A61B 90/00

(54) **A SYSTEM FOR DETECTING TISSUE FLUID CHANGES**

(71) Applicant: Nagravision S.A., 1033 Cheseaux-sur-Lausanne (CH)
(72) Inventor: Villegas, Karine, 1033 Cheseaux-sur -Lausanne (CH); Keomany, Jean, 1033 Cheseaux-sur -Lausanne (CH); Perrine, Jérôme, 1033 Cheseaux-sur -Lausanne (CH)
(74) Representative: Hoyng Rokh Monegier B.V.

(57) **Abstract**

Providing methods and systems for use in monitoring and detecting changes in the level of fluid.

## Description

### FIELD OF THE INVENTION

The present disclosure generally relates to methods and systems for use in monitoring and detecting changes in the level of fluid and/or other material in a living tissue, more particularly to methods and systems for use in monitoring and detecting edema.

### BACKGROUND

Elevated or abnormal fluid levels in living tissue can result from a number of physiological conditions. For example, edema is an abnormal accumulation of fluid in the intercellular spaces of connective tissue. Edematous tissues are swollen and when punctured secrete an incoagulable fluid. Edema is a symptom of disease and caused generally due to alteration in the physiological mechanisms that maintain a constant water balance in the cells, tissues, and blood. Among the causes may be diseases of the kidneys, veins, or lymphatic system; surgery; or chemotherapy. Because dependent edema is a physical indication of a number of different disease conditions, the development of accurate methods for the detection of edema is of interest.

Edema may be detected by visits to medical-care providers, such as physicians, nurses and massage therapists. However, frequent visits to medical-care providers are inconvenient to the patient.

It is possible to place various electronic devices on a patient to detect or monitor edema condition. For example, pressure sensors may be attached to the patient's limb by bandages to monitor the edema condition; if swelling occurs, the pressure sensors will detect the corresponding changes to the pressure exerted by elasto-compression. The pressure sensors are wired to an electronic device that may receive and analyze the signals produced by the pressure sensors.

The existing methods require patients to be physically connected to electrical devices. For examples, sensors are normally placed on patients' skin. This significantly disturbs the patient's daily life. In addition, detection accuracy may be reduced due to pressure change excreted on the sensors, for example, when the bandage becomes loose or the sensors become displaced.

In view of the serious health issues caused by abnormal accumulation of fluid in living tissue, and the extreme importance of its early detection, it would be desirable to provide devices, systems and methods for use in monitoring and/or detecting changes in the level of fluid and/or other material in a living tissue.

It is desirable to develop more accurate devices, systems and methods for detecting changes in fluid levels in living tissue, and particularly, for monitoring and/or detecting elevated or otherwise abnormal levels of fluids in living tissue, for example, as a result of edema.

It would also be desirable to provide methods and systems for use in monitoring and/or detecting changes in the level of fluid and/or other material in a living tissue that reduce the need for frequent visits to medical-care providers, are easy to use, that minimizes or avoids connecting electronic devices to the patient and improve patient's daily life.

The present invention provides such devices, systems and methods that solve one or more of the problems mentioned above. Other features and advantages of the invention will be apparent from the following description and from the claims.

### BRIEF DESCRIPTION OF THE DRAWINGS

The features and advantages of the invention will be appreciated upon reference to the following drawings, in which:
Fig. 1 illustrates an exemplary environment in which a method and an image-capturing device in accordance with an embodiment of the present disclosure may be applied.
Fig. 2 illustrates two exemplary images acquired by an exemplary image-capturing device in accordance with an embodiment of the present disclosure.
Fig. 3 illustrates two exemplary images acquired by an exemplary image-capturing device in accordance with an embodiment of the present disclosure.
Fig. 4 illustrates an exemplary image-capturing device in accordance with an embodiment of the present disclosure.
Fig. 5 shows a hardware infrastructure for implementing an embodiment.

The figures are meant for illustrative purposes only, and do not serve as restriction of the scope or the protection as laid down by the claims.

### DESCRIPTION OF ILLUSTRATIVE EMBODIMENTS

The devices, computer programs, systems, and methods of the present disclosure are applicable to the monitoring any abnormal fluid accumulation within a living tissue (whether a body fluid or an introduced fluid). One skilled in the art will appreciate that elevated, abnormal or changing levels of generally any fluid can be monitored and detected using the devices, computer programs, systems and methods of the present disclosure. Detection of changes in level of fluids in the living tissue includes, but is not limited to, the detection of fluid changes as a result of edema, ruptured organ and the like.

In one aspect of the present disclosure, a method for monitoring and/or detecting changes in a level of fluid and/or other material in a living tissue is provided. The method comprises processing a digital image data set obtained from an artificial image placed on a living tissue to extract one or more parameters from the artificial image; comparing the extracted one or more parameters with a reference model; determining whether the one or more parameters of the artificial image exceeds or is within a threshold value predetermined by the reference model based on comparison of one or more parameters of the artificial image and the reference model; and generating an output comprising an indication of change in the level of fluid in living tissue, when the one or more parameters of the artificial pattern exceeds the threshold value.

The one or more parameters of artificial image includes any predetermined feature of the artificial image that is detectable by an image processing and analysis software or image-capturing device known to a person skilled in the art. For example, the one or more parameters comprise at least one from the group consisting of contour of the artificial image, orientation of the artificial image, shape of the artificial image, stretch of the artificial image, circumference of the artificial image, volume of the artificial image, 2D and/or 3D image of the artificial image, swelling of the artificial image, and color of the artificial image.

The term "artificial image" used herein means an image drawn by a computer or a hand drawn image. Examples of the artificial image include, for example, a letter, a geometric line, an illustration, and a logo, etc. The artificial image can be drawn, implanted, tattooed, attached, adhered to a patient by any known method. The artificial image does not refer to naturally occurring images produced via biological mechanism of a patient, such as moles, rashes, and the like.

The term "patient" refers to any individual (human or animal) with respect to whom an artificial image is intended to be, or has been, used. Optionally, the patient is human.

In an embodiment, the artificial image is a tattooed to the patient, preferably, the tattoo is erasable or is a temporary tattoo. A tattoo may be applied to the patient's body by known methods. A permanent tattoo is robust against wearing off, which may be useful if the target living tissue has to be monitored for a long term. Alternatively, an erasable tattoo may be more applicable if the patient does not want permanent changes to the appearance of the body (e.g. skin) to be monitored. Other forms of the artificial pattern are possible, such as a sticker.

The artificial image of present disclosure (e.g. the pattern of the artificial image) is designed that when the level of fluid and/or other materials in living tissue, which is in contact or is in proximity with the artificial image exceeds a predetermined threshold as set by the reference model, at least one of the predetermined parameters of the artificial image changes.

In an embodiment, the artificial image is invisible under visible light and preferably visible under UV light. This may reduce or eliminates the impact of the artificial pattern on the patient's appearance. Images acquired by an ordinary camera cannot show such artificial image, but specialized image-capturing devices can. The artificial pattern may be alternatively or additionally visible to other spectrum, such as infrared. In an embodiment, the artificial pattern is removable from the skin. This avoids permanent impact to the patient's appearance.

In an embodiment, the artificial image is covered by a material that is at least partly transparent, preferably a transparent plaster. This may be suitable if the skin or tissues to be monitored have to be protected.

In an embodiment, the artificial image comprises a plurality of sub-patterns, wherein the measuring one or more parameters of the artificial pattern in the acquired image comprises measuring one or more parameters of at least some of, optionally each of, the sub-patterns. The sub-patterns may allow the monitoring of a larger area or provide more detailed information within the same area.

In an embodiment, the comparing of the extracted and measured one or more parameters of the artificial image in the acquired image with the reference model comprises comparing the extracted and measured one or more parameters of sub-patterns with a predetermined one or more parameters of a respective sub-pattern of the artificial pattern (i.e. the reference model). The comparison of the sub-patterns may provide focused monitoring.

In an embodiment, the sub-patterns carry a weight in the comparing of the measured one or more parameters of the artificial image in the acquired image with the predetermined one or more parameters of the artificial pattern (i.e. reference model). The weighting of the sub-patterns may allow emphasis of a particular area (e.g., one that is medically critical or representative) and/or de-emphasis of an area that is less important or more error prone. In an embodiment, the weight is associated with a location of the respective sub-pattern and/or with prior results of the comparison. For example, some sub-patterns may be de-weighted if the edema condition around these sub-patterns has been stable. Moreover, other sub-patterns may get an increased weight if known to be more critical, more sensitive or more resistant to treatment.

In an embodiment, the comparing of the extracted and measured one or more parameters (e.g. shape) of the artificial pattern in the acquired image with the reference model comprises: determining whether a difference between the extracted and measured one or more parameters and the reference model exceeds a threshold. The threshold may help filter out minor changes not attributable to the underlying change in level of the fluid in living tissue. Such minor changes may be due to, e.g., electronic noises in the image-capturing device and the user's handshaking when taking the pictures. The threshold value may vary and updated as often as necessary before the artificial image is placed on a patient or during a period when the artificial image is being used by the patient. The variation or update of the threshold value can depend on, for example, patient-specific parameters, i.e. specific for each patient receiving the artificial image as will be explained below.

The term "proximity" is defined as sufficiently close to the artificial image such that a change in level of fluid or other materials in living tissue will cause a change in one or more parameters of the artificial image. For example, if the artificial pattern is applied on the patient's palm of his right hand, then not only are the tissues under the palm are considered in proximity to the artificial pattern but also the fingers may be considered in proximity to the artificial pattern provided that the change to the fluid level of the fingers cause at least one parameter of the artificial pattern to change. A more detailed example is that, if the swelling (because of, e.g., inflammation or excessive cellular fluid accumulation) in the lower part of a finger of the right hand is large enough to cause the shape of the artificial pattern on the palm to change, then said lower part of said finger is considered in proximity with the artificial pattern; on the other hand, the left hand is normally not considered as in proximity with the artificial image placed on the right hand (unless the condition on the left hand can affect the shape of that artificial image).

The term "living tissue" used herein refers to a biological tissue (also including an organ) which is not fixed or frozen at the time of contact by, and labeling with, the artificial image.

As used herein the term "reference model" refers to predetermined and desired model, sub-model, values, standards, threshold values, or parameters available to a service provider such as medical professionals, manufactures of the artificial image, or developer of a particular artificial image. For example, the artificial image developer sets certain standard values for the life of the artificial image, color fade or wash of the image, i.e. wear of the artificial image, line spacing, pattern, shape and (re)application recommendations and/or rules in the reference model. The reference model can be used here to model the (e.g. via algorithm) behavior of the artificial image for the purpose of model-based image diagnoses and/or monitoring for use in detecting and/or monitoring a change in a level of fluid in living tissue. The reference model is designed specifically for a particular image and optionally for a particular patient and/or living tissue that the artificial image is intended. The reference model can be changed, updated, and/or optimized before or during the use of the artificial image.

In one embodiment, the reference model of present disclosure in addition to comprising predetermined values of the artificial image, it also comprises at least one patient-specific's parameter.

Additional patient-specific measurements and information that can be used in creating the reference model can include, for example, soft and connective tissue structures of the patient, skin color of the patient, skin texture of the patient, muscle tone of the patient, and patient's personal and medical information, such as patient's age, weight, gender, ethnicity, activity level, and medical conditions such as allergies, skin diseases.

In addition to (or optionally in place of) the above-mentioned measurements, it may be desirable to have the reference model to include measurements (one or more parameters) of the artificial pattern as well as surrounding anatomical areas of the patient's anatomy. Such measurements can provide, for example, data on movement of artificial image, which can be prone to more wear or stretch marks.

Optionally, the reference model is generated (e.g. its threshold value has been determined) based on the one or more parameters of the artificial pattern, optionally, it can be generated by combination of the one or more parameters of the artificial pattern and patient-specific parameter before it is placed on the living tissue or during use by the patient.

As used herein the term "patient-specific parameter" refers to a variety of information which is unique to each patient having or receiving the artificial image. For example, the patient-specific information includes family medical history information, medical conditions (e.g. allergy information, diseases,), exercise information, drug prescriptions, skin and body conditions (e.g. skin type, skin texture, muscle tone, etc.), age of the patient or any combinations of any of the patient-specific parameter. Optionally, in the reference model, the one or more parameters of the artificial image are normalized with a patient-specific information.

Optionally, the reference model is stored in at least one remote element (e.g. server), in an image-capturing device (e.g. smartphone or camera) or in a non-transitory computer readable storage medium according to the present disclosure.

In an embodiment, the method of present disclosure comprises generating a signal in response to a result of the comparison of the extracted and measured one or more parameters of the artificial pattern in the acquired image and the reference model. For example, if the result of the comparison indicates that the fluid level of living tissue that is being monitored exceeds a threshold level as set in a reference model, a signal in the form of an alarm may be generated. The signal could attract attention of the patient and/or the medical-care provider. A signal may also be generated when the comparison indicates that the physiological condition being monitored has been stable.

In an embodiment, the signal is a visual, audio, or tactile alert. A visual signal may be in the form of a warning message on a screen of an electronic device of the patient or the medical-care provider. A visual signal may also be in the form of blinking lights emitted from, e.g., an LED on the patient's smartphone. An audio or tactile signal may be useful in situations such as when the patient or the medical-care provider has fallen asleep but urgent medication attention is required.

In an embodiment, the acquiring of an image of the artificial image on the skin, the extracting and measuring one or more parameters of the artificial image, and the comparing of the measured one or more parameters of the artificial image with the reference model are each performed a plurality of times. Repeated measurements and analysis may increase the monitoring accuracy. The measurements and analysis may be averaged out to allow the trend to show or be filtered to remove the effects of short-term disturbances.

In an embodiment, the frequency at which the acquiring, the measuring and the comparing is performed depends at least in part on one or more of the previously measured one or more parameters (e.g. shapes).This may allow optimization of the attention needed from the patient and/or the medical-care provider, giving them more convenience. For example, if prior measurements indicate a stabilizing condition, the patient may take fewer images of the artificial pattern per day and have less disruption to his daily activities.

In an embodiment, the acquiring, the measuring and the comparing are performed at regular periods; or wherein the acquiring, the measuring and the comparing are performed on-demand.

Performing such actions at regular periods, instead of once, is often useful in effectively monitoring the physiological condition. The periods may be measured in seconds, minutes, hours or days. For example, a period between 2 hours and 4 hours may be considered a practical balance between monitoring frequency and disturbance to the patient. It is of course possible that different actions may be taken at different periods. For example, the acquisition of the image of the artificial pattern, which often requires the patient's action (e.g., taking his smartphone to take a picture), may be performed less frequently than the measurement of the shape and the comparison.

Alternatively or additionally, the acquiring, the measuring and the comparing may be performed on-demand based on, e.g., the patient's feeling of his own health, the medical-care provider's instruction, or the recommendation from software that analyzes the patient's historical data.

In another aspect of the present disclosure, a non-transitory computer readable storage medium is provided.

The term "non-transitory computer-readable medium" as used herein refers to any medium that comprises the actual performance of an operation (such as hardware circuits), that comprises programs and/or instructions to be provided to one or more processors for performance/implementation (such as instructions stored in a non-transitory memory), and/or that comprises instructions stored in memory. Non-transitory computer-readable media may take many forms, such as nonvolatile and volatile media, including but not limited to, a floppy disk, flexible disk, hard disk, RAM, any memory chip, any magnetic medium from which a computer instruction can be read; a CD-ROM, DVD, or any other optical medium from which a computer instruction can be read, or any other non-transitory medium from which a computer instruction can be read.

In one embodiment, the programs and/or higher-level instructions in the non-transitory computer-readable medium intend to be used for one or more medical purposes such as detecting or monitoring edema. Optionally, the programs and/or higher-level instructions in the non-transitory computer-readable medium intend to be used without being part of a hardware medical device. "without being part of' means software not necessary for a hardware medical device to achieve its intended medical purpose.

In one embodiment, the non-transitory computer readable storage medium is used for detecting and/or monitoring a change in a level of fluid in living tissue, optionally, for use in detecting and/or monitoring edema. The non-transitory computer readable storage medium is configured to store instructions that when executed cause a processor to perform comparing data representative of one or more parameters extracted from an artificial image placed on a portion of a living tissue (e.g. contour of a tattooed image on or underneath of patient skin) with a reference model.

In one embodiment, the non-transitory computer-readable storage medium according to present disclosure is configured to store instructions that when executed cause a processor to perform comparing data representative of one or more parameters extracted from the artificial image which is placed on a portion of a living tissue with a reference model; wherein the instruction configured that when executed to cause the processor to detect and/or to monitor whether the extracted one or more parameters of the artificial pattern exceeds and/or is within a threshold value predetermined by the reference model based on comparison of the extracted one or more parameters of the artificial pattern and the reference model and generating an alert output when the one or more parameters of the artificial pattern exceeds the predetermined threshold value. When the one or more parameters of the artificial pattern exceeds the predetermined threshold value, this is indicative that the level of fluid in the living tissue has been changed or it is changing.

Optionally, the non-transitory computer-readable storage medium according to any of the paragraphs is configured to store instructions that when executed cause a processor to receive data representative of the reference model, which is predetermined based on one or more parameters of that specific artificial image placed or to be placed on the portion of the living tissue, alternatively or additionally, the reference model is predetermined based at least on one patient-specific parameter such as parameters of the specific portion of living tissue and/or patient receiving the artificial image (patient-specific parameter). The one or more parameters that is used to create the reference model serve can be served as the threshold value for use in the comparison as disclosed in above and to create an output.

The non-transitory computer-readable storage medium of any of the paragraphs, wherein the non-transitory computer-readable storage medium configured to store instructions that when executed cause a processor to compare at least one extracted parameter during a first period of time with a reference model and to compare at least one extracted parameter during a second period of time with a reference model; generating an output indicative progression of change in one or more parameters of the artificial image. The output is indicative whether level of fluid in the living tissue has been increased, maintained, or reduced.

One skilled in the art will appreciate that any of paragraphs of the present disclosure such as paragraphs to the reference model and the one or more parameters can be applied and it is incorporated by reference in its entirety to paragraphs of the non-transitory computer-readable storage medium.

In another aspect of present disclosure an image-capturing device comprising a processor and, optionally, a non-transitory computer-readable storage medium according to any of the paragraphs is provided. The image-capturing device of present disclosure is for use in detecting and/or monitoring a change in a level of fluid in living tissue, optionally for use in detecting and/or monitoring edema.

The image-capturing device of the present disclosure is located outside the patient and it is configured to perform at least one from a the following functions: to take a photographic image of the artificial image; to extract one or more parameters of the artificial image; to store a reference model; to store and update the reference model when needed; to compare the extracted one or more parameters of the artificial image with a reference model; to detect and/or monitor change in one or more parameters of the artificial image; to produce a measurable output; and to produce an output indicative a change in a level of fluid in living tissue when the extracted one or more parameters of the artificial image exceeds the predetermined threshold values as set in the reference model. Optionally, the output of the image-capturing device may be measured and displayed by various types of consumer electronics devices, including, but not limited to, personal computers, digital video disk devices, television sets, audio reproduction systems, smart phones, and notebooks. However, in various alternate embodiments, any appropriate electronic network designed to permit communication between any desired types of electronic devices.

In one embodiment, acquiring an image of the artificial pattern does not necessarily require the attention of medical-care providers, nor does it necessarily involve attaching and wiring sensors to the skin or tissues to be monitored. Therefore, the physiological condition may be monitored without frequent visits to the medical-care providers. Moreover, disturbances to the patient's daily life are reduced.

For example, the predetermined shape of the artificial image may be associated with the condition of no swelling, thereby allowing detection of any swelling. The predetermined shape may also be associated with a certain amount of swelling; in this case, changes to the shape of the artificial pattern may indicate a deterioration of edema (further swelling) or an improvement. If the improvement is caused by administration of medication, the efficacy of the medication and the recovery speed may also be monitored.

In another embodiment, patients treatment and medications can be monitored via the paragraphs of present disclosure. For example, certain medication may cause the chemical composition in the patient's sweat to change. If the color of the artificial pattern is responsive such chemical composition (e.g., by using a special ink that changes color in response to chemical changes in the surrounding environment), the existence of such medication can be monitored through images of the artificial image. With sufficient sensitivity of the color of the artificial image, the dosage or concentration of such medication can also be monitored through images of the artificial pattern without frequent visits to clinics or labs or wired sensors attached to the patient.

In an embodiment, the image of the artificial pattern on the skin is acquired by an image-capturing device, for example, a camera or a mobile device comprising a camera. The image-capturing device may be convenient to the patient to acquire images of the artificial pattern. This is especially true given the prevalence of hand-held cameras that are easy to use and carry as well as mobile devices that comprise a camera. An example of such mobile devices is a smartphone. The ubiquity of smartphone obviates the need for a special-purpose image-capturing device, adding to the patient's convenience.

In an embodiment, the image-capturing device is communicatively coupled to a processing device (i.e. a remote device), optionally, the processing device is not in physical contact with the image-capturing device. The processing device may augment the image-capturing device in non-image-capturing functions, such as large-scale storage and analysis of image data. The processing device may be, e.g., the computer of a physician or a remote server.

In an embodiment, the image-capturing device comprises a wireless communication module, a secure communication module, or both. The wireless communication module may make it easy for the patient to communicate image and/or analysis data with, e.g., a physician or a nurse. Since the data may contain sensitive personal and/or medical information, a secure communication module could strengthen the patient's privacy.

In an embodiment, the secure communication module ensures confidentiality and/or integrity and/or authenticity of the data transmitted from or received by the image-capturing device. Data confidentiality protects the patient's privacy. Integrity ensures that the data in transit are not corrupted or tempered with. Authenticity ensures, e.g., the data received by the physician indeed come from the correct patient.

In an embodiment, the processor comprises a computation module, preferably a CPU; wherein the computation module compares the measured and extracted one or more parameters of the artificial image in the acquired image with a predetermined reference model of the artificial image. In one embodiment, the computation model is coupled with the non-transitory computer readable storage medium of present disclosure.

In one embodiment, the image-capturing device comprises a computation module. With such computation module, the image-capturing device can make the comparison itself without relying on a remote device (e.g. a server). For example, a smartphone may acquire the image and perform the processing at the same time to provide instant feedback to the patient. The smartphone may also communicate the comparison to a remote device, such as a physician's computer.

In an embodiment, the computation module is configured to receive algorithmic training from a remote server, the algorithmic training relating to the measuring the one or more parameters of the artificial pattern in the acquired image (e.g. shape) and/or the comparing of the measured one or more parameters of the artificial pattern in the acquired image with the reference model.

The computation module enables the image-capturing device to process image data locally, instead of having to rely on a remote server. However, a remote server can also be used to perform measurement and image analysis and comparison according to the present disclosure. For example, the computing power of the image-capturing device may be limited because of its size or portability. This limitation may make it less feasible to implement some algorithms or algorithmic training that improve the performance of image data processing but often require large computational power. The remote server that provides algorithmic training to the image-capturing device may obviate this limitation.

It is also possible that the computation module of the image-capturing device itself possesses sufficient computational power to locally train the image processing algorithms, such as shape measurement and comparison.

In an embodiment, the image-capturing device pushes at least one of its functions to another remote device such as server or smartphone of medical practitioner.

Pushing is a form of proactively communicating data from the image-capturing device to another device. This may reduce delay in situations where further actions by the patient or the physician depend on information reaching said another device. For example, the physician may not be up-to-date with the patient's condition without the latest data. Pushing the acquired image to the processing device may accelerate the storage and further analysis of the acquire image. Pushing the result of the comparison may create an early alert to the physician or nurse remotely monitoring the patient.

In an embodiment, the computational module detects a trend from the comparison between the extracted one or more parameters of the artificial images in a plurality of acquired images with the reference model. This allows continuous monitoring of the target living tissue and fluid level. It also enables monitoring progress of the medication that treats the target living tissue such as edema.

In another aspect of present disclosure, a server device is provided. The server is for use in detecting and/or monitoring a change in a level of fluid in a living tissue, optionally for use in detecting and/or monitoring edema The server device comprises a data receiving device such as a transceiver configured to receive data representative of the extracted one or more parameters of the artificial image placed on a living tissue generated during a time period by the image-capturing device of present disclosure; a processor operatively coupled to the transceiver; and one or more non-transitory computer readable storage medium according to any of the paragraphs operatively connected to the processor.

In this embodiment, it is clear that any of the paragraphs of the present disclosure is incorporated by reference to the embodiments representing the server. For example, all the paragraphs of present disclosure related to a non-transitory computer readable storage medium configured to store instructions that when executed cause a processor to perform any functions mentioned above alone or in combination; the image capturing device are incorporated by reference.

According to an aspect of the present invention, there is provided a system for use in detecting and/or monitoring a change in a level of fluid in a living tissue, optionally for use in detecting and/or monitoring edema, said system comprises the image-capturing device, the non-transitory computer-readable medium, the server according to any of the paragraphs of present disclosure. Optionally, the system comprises the artificial image of present disclosure. Optionally, the system comprises a remote device for receiving the output from the image-capturing device. Optionally, the system further comprising: a pattern applicator adapted to apply the artificial pattern on the skin.

In one embodiment, the system comprises: a server configured to receive a digital image data representative of one or more parameters extracted from the artificial image placed on a living tissue sent from the image-capturing device of present disclosure; a processor, the processor operable for: receiving the digital image data set from the image-capturing device; processing the digital image data set to extract one or more parameters from the image; comparing the extracted one or more parameters with a reference model (e.g. predefined one or more parameters of the artificial image) and generating an output comprising an indication of change in the level of fluid in the living tissue. Optionally, the process is configured to be coupled to the non-transitory computer-readable storage medium according to any of the paragraphs.

The non-transitory computer readable storage medium; servers; systems; devices; and methods according to any of the paragraphs of present disclosure address the problem of fluid accumulation in a living tissue. They capture change in fluid level of living tissue outside of the clinical settings and enable the patient engagement. Unlike current procedures of connecting electrical devices to patients and frequent visits of patients to professional healthcare providers, the present disclosure enables patients to monitor and to detect edema at very early stage by monitoring their acritical image as frequent as needed without a need to visit professional every time. Furthermore, the analysis can be modified based on responses and medical conditions of patients.

For example, the non-transitory computer readable storage media; servers; systems; medical devices; and methods according to any of the paragraphs of present disclosure are able to monitor/screen/detect a disease or condition via detecting a change in a level of a fluid in living tissue, to identify early signs of a disease or conditions, to inform the patient and medical practitioners about the available options to reduce or to prevent fluid accumulation, and to provide clinical information by aggregating relevant information.

The detailed description set-forth above is provided to aid those skilled in the art in practicing the present invention. However, the invention described and claimed herein is not to be limited in scope by the specific embodiments herein disclosed because these embodiments are intended as illustration of several aspects of the invention. The embodiments set-forth above can be performed and combined with other disclosed embodiments according to the invention. Any equivalent embodiments are intended to be within the scope of this invention. Indeed, various modifications of the invention in addition to those shown and described herein will become apparent to those skilled in the art from the foregoing description which do not depart from the spirit or scope of the present inventive discovery. Such modifications are also intended to fall within the scope of the appended claims. All publications, patents, patent applications and other references cited in this application are incorporated herein by reference in their entirety for all purposes to the same extent as if each individual publication, patent, patent application or other reference was specifically and individually indicated to be incorporated by reference in its entirety for all purposes. Citation of a reference herein shall not be construed as an admission that such is prior art to the present invention.

It should be evident that each individually recited features may be combined with any number of other individually recited feature(s), absent any obvious contradiction.

### EXAMPLES

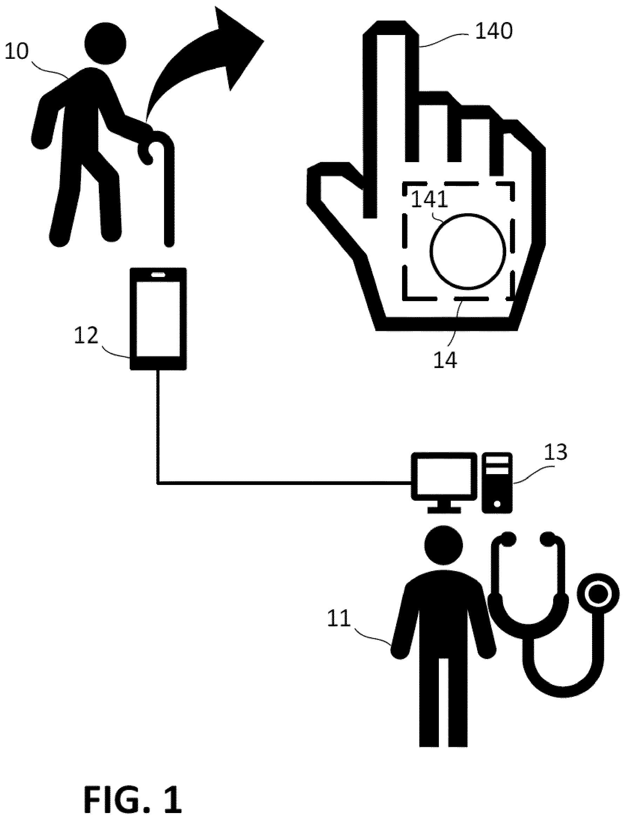

Fig. 1 illustrates an exemplary environment in which a method and an image-capturing device in accordance with an embodiment of the present disclosure may be applied. There is a patient 10 and a medical-care provider 11, such as a physician, who may want to detect and/or monitor change in fluid level of a living tissue area of the patient 10 and perform treatments if necessary. The patient 10 possesses an image-capturing device 12. The physician 11 may have a processing device 13, such as a desktop, a laptop, a mobile phone or a tablet. The patient 10 may use his image-capturing device 12 to acquire pictures of the living tissue area that is being monitored. In the example illustrated in Fig. 1, the living tissue area that is being monitored is on his right hand 140. There is an artificial image 141 on the right hand 140 and its usage will be discussed in more detail.

Monitoring and/or detecting change in fluid level of living tissue of the patient 10 may require regular or periodic monitoring. An example is tissue or limb swelling caused by inflammation or edema, which, if left unattended, often result in undesirable medical consequences.

The physician 11 may inspect the patient 10 to monitor the level of fluid in the target living tissue. Requiring the patient 10 to visit the physician's office for every inspection is burdensome.

Instead, the patient 10 may use his image-capturing device 12 to take an image 14 of his artificial image 141. The image-capturing device 12 may analyze (e.g. extract one or more parameters, optionally, compare them with a reference model) the image 14 and, depending on the analysis result, generate a signal to notify the patient 10 or the physician 11. The image-capturing device 12 may also send the image to the processing device 13 for the physician 11 to review. The processing device 13 may also analyze the image 14 prior to the physician's review and, depending on the analysis result, generate a signal.

The image-capturing device 12 may be a camera or a mobile device comprising a camera, such as a smartphone or a tablet. The image-capturing device 12 may comprise a image acquisition module, a computation module (such as a central processing unit, CPU), a communication module and a memory.

The image-capturing device 12 is communicatively coupled to the processing device 13. The communicative coupling may be wired or wireless. The communicative coupling may be secure to ensure the confidentiality, integrity and/or authenticity of the data communicated between the image-capturing device 12 and the processing device 13.

The processing device 13 does not have to be associated with the physician 11. For example, the processing device 13 may be a remote server of a third-party provider that offers services such as medical data analysis and management.

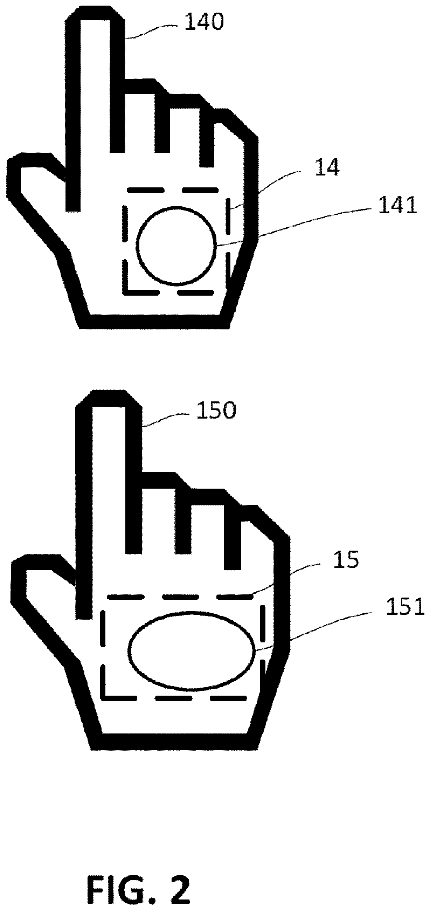

A more detailed explanation of the present disclosure will be made by referring to Fig. 2, which illustrates two exemplary images 14 and 15 acquired by the image-capturing device 12.

The images 14 and 15 are acquired at different times. The image 14 shows the hand 140 with the pattern 141. When the image 15 is taken, there have been changes to the level of fluid in the tissue, making the hand 150 swell and become bigger than the hand 140 in the image 14. The pattern 151 in image 15 also changes its shape. In the example of Fig. 2, the pattern 151 has an expanded shape compared to the pattern 141 in the image 14.

The shapes of the patterns 141 and 151 in images 14 and 15 may be measured. The measurement may be performed by the image-capturing device 12 itself; or the images 14 and 15 may be sent to another computer for the measurement. The patterns 141 and 151 in images 14 and 15 may optionally be detected first and separated from the other objects in the images and 15 (such as the patient's hands 140 and 150), prior to the measurement of the shapes of the patterns 141 and 151.

The measured shapes of the patterns 141 and 151 are then compared to each other. Likewise, the comparison may be done by the software in the image-capturing device 12 itself or in another computer communicatively coupled to the image-capturing device 12, such as the processing device 13 illustrated in Fig. 1.

Compared to the shape of the pattern 141, the shape of the pattern 151 may indicate that there has been swelling, which may be caused by a worsening edema condition. Depending on the amount of difference, a signal may be generated to notify the patient 10 or the physician 11. The signal may take the form of a visual, audio, or tactile alert. The visual alert may be in graphical forms (such as an alarm icon on the patient's image-capturing device 12) or in texts (such as an email sent to the physician's email account).

In an embodiment, the difference between the shapes of the patterns 141 and 151 is quantified. The signal may be generated only if the quantified difference exceeds a threshold. The threshold may vary according to the patient's medical condition and any material that is close to or covers the artificial pattern.

The difference in time between when the images 14 and 15 are taken may be regular, such as every 0.5, 1, 2, 3, 4, 5, 6, 7, 8, 9 or 10 hours. The difference in time between when the images 14 and 15 are taken may depend on the patient's medical history and/or the physician's judgement.

Although Fig. 2 illustrates that two images are taken, taking more images is certainly possible, as are multiple measurement of the shape of the artificial pattern and multiple comparison between/among the measured shapes. The images may be acquired more or less frequently depending on previous comparison results. For example, improving edema condition may warrant less frequent image acquisition and subsequent shape measurement and comparison.

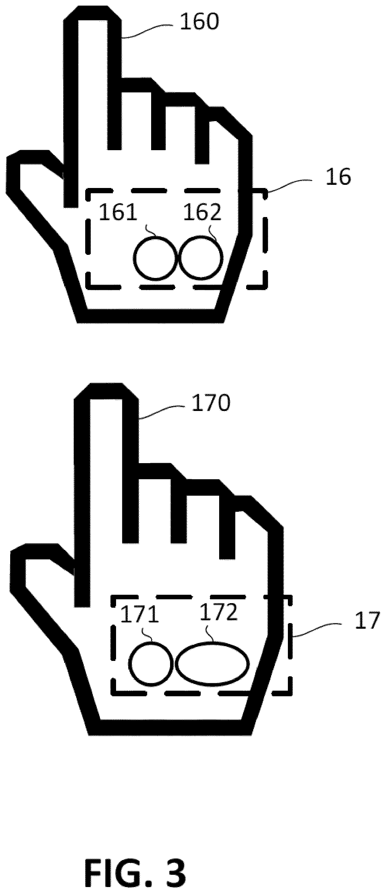

Fig. 3 illustrates two exemplary images 16 and 17 acquired by an image-capturing device and represents a variation to Fig. 2.

In Fig. 3, the artificial image comprises two sub-patterns 161 and 162. The sub-patterns may or may not contact each other. The sub-patterns may respond differently to the change in level of fluid of different living tissues areas.

The sub-patterns may allow more refined monitoring of the physiological condition. In the example of Fig. 3, the hand 170 in the image 17 generally swells compared to the hand 160 in the image 16. However, the difference between the sub-patterns 161 and 171 is less than that between the sub-patterns 162 and 172. This may indicate, for example, that the edema condition below the ring and little fingers is more serious than below the middle finger.

In an embodiment, each sub-pattern may carry a different weight during the comparison. The weight may be associated with a location of the respective sub-pattern and/or with prior results of the comparison of the respective sub-pattern.

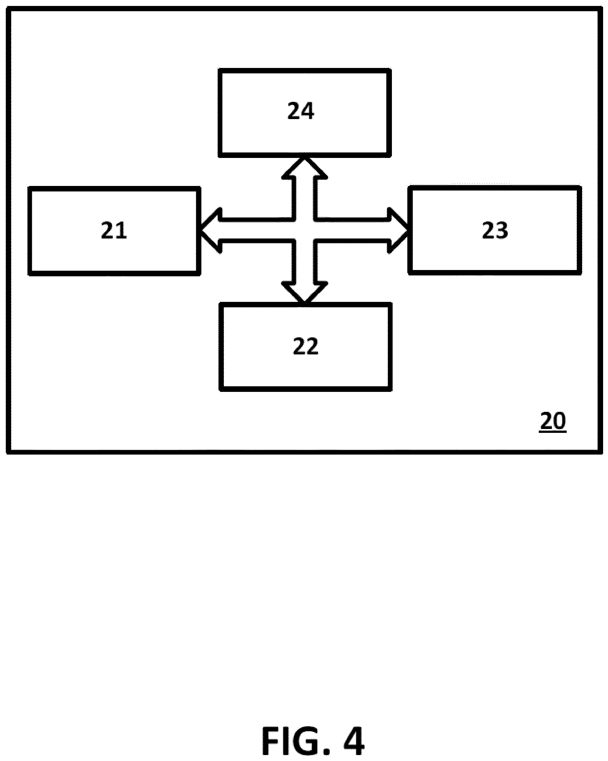

Fig. 4 illustrates an exemplary image-capturing device 20 in accordance with an embodiment of the present disclosure.

The image-processing device 20 comprises an image acquisition module 21, a computation module 22, a communication module 23 and a memory 24. As shown in the cross arrow in Fig. 4, the modules 21, 22, 23 and the memory 24 may be communicatively (e.g., electronically) coupled to each other. The image-processing device 20 may be a camera or a smart mobile device.

The image acquisition module 21 may comprise a lens, light sensors and color sensors. The image acquisition module 21 may be configured to acquire images. The image acquisition module 21 may be a camera in a smartphone. As is popular nowadays, there may be more than one image acquisition modules 21 in the image-processing device 20.

The computation module 22 may a central processing unit (CPU). The computation module 22 may be configured to process the images acquired by the image acquisition module 21, such as detecting and measuring the shape of various objects in the acquired images and comparing among the measured shapes. The computation module 22 may be configured to process multiple images and detects a trend in the comparison results. The computation module 22 may be configured to receive algorithmic training. The training may be based on pattern recognition training models. The training may be based on artificial-intelligence techniques.

The communication module 23 may be configured to transmit data from the image-processing device 20 and receive data to the image-processing device 20. The communication module 23 may provide wired and/or wireless communication capabilities. The communication module 23 may also provide secured communication capability to protect the confidentiality, integrity and authenticity of the data. There capabilities may be implemented within the same module (e.g., as in different software blocks at the logical layer) or as different sub-modules (e.g., transceiver, modem, encryption/decryption engine).

The communication module 23 may be configured, at the request of the computation module 22 for example, to push various information to a remote processing device. The information that can be pushed includes images acquired by the image acquisition module 21, shapes of various objects in the acquired images measured by the computation module 22, and the result of the comparison by the computation module 22.

The memory 24 may store various information. One information that is useful to store in the memory 24 is the predetermined shape of the artificial pattern. Such predetermined shape may serve as a baseline for some or all of the measurements, comparison and computation performed by the computation module 22.

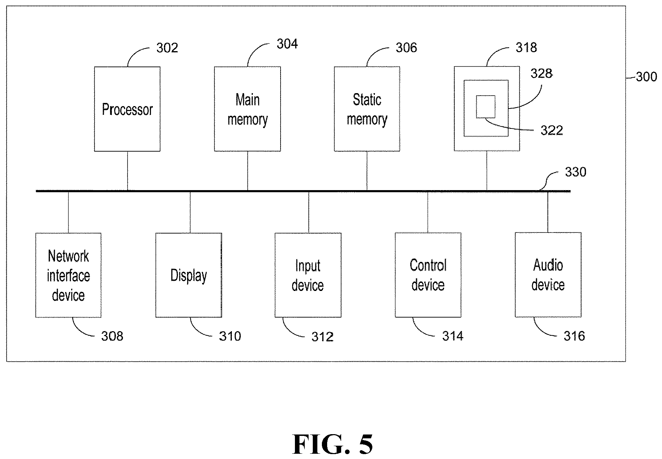

Figure 5 illustrates a block diagram of one implementation of a computing device 300 within which a set of instructions, for causing the computing device to perform any one or more of the methodologies discussed herein, may be executed. The computing device 300 may be used for elements of a system used to carry out the present disclosure.

In alternative implementations, the computing device may be connected (e.g., networked) to other machines in a Local Area Network (LAN), an intranet, an extranet, or the Internet. The computing device may operate in the capacity of a server or a client machine in a client server network environment, or as a peer machine in a peer-to-peer (or distributed) network environment.

The computing device may be a personal computer (PC), a tablet computer, a set-top box (STB), a wearable computing device, a Personal Digital Assistant (PDA), a cellular telephone, a web appliance, a server, a network router, switch or bridge, or any machine capable of executing a set of instructions (sequential or otherwise) that specify actions to be taken by that machine. Further, while only a single computing device is illustrated, the term "computing device" shall also be taken to include any collection of machines (e.g., computers) that individually or jointly execute a set (or multiple sets) of instructions to perform any one or more of the methodologies discussed herein.

The example computing device 300 includes a processing device 302, a main memory 304 (e.g., read-only memory (ROM), flash memory, dynamic random access memory (DRAM) such as synchronous DRAM (SDRAM) or Rambus DRAM (RDRAM), etc.), a static memory 306 (e.g., flash memory, static random access memory (SRAM), etc.), and a secondary memory (e.g., a data storage device 318), which communicate with each other via a bus 330.

Processing device 302 represents one or more general-purpose processors such as a microprocessor, central processing unit, or the like. More particularly, the processing device 302 may be a complex instruction set computing (CISC) microprocessor, reduced instruction set computing (RISC) microprocessor, very long instruction word (VLIW) microprocessor, processor implementing other instruction sets, or processors implementing a combination of instruction sets. Processing device 302 may also be one or more special-purpose processing devices such as an application specific integrated circuit (ASIC), a field programmable gate array (FPGA), a digital signal processor (DSP), network processor, or the like. Processing device 302 is configured to execute the processing logic (instructions 322) for performing the operations and steps discussed herein.

The computing device 300 may further include a network interface device 308. The computing device 300 also may include a video display unit 310 (e.g., a liquid crystal display (LCD) or a cathode ray tube (CRT)), an alphanumeric input device 312 (e.g., a keyboard or touchscreen), a cursor control device 314 (e.g., a mouse or touchscreen), and an audio device 316 (e.g., a speaker).

The data storage device 318 may include one or more machine-readable storage media (or more specifically one or more non-transitory computer-readable storage media 328) on which is stored one or more sets of instructions 322 embodying any one or more of the methodologies or functions described herein. The instructions 322 may also reside, completely or at least partially, within the main memory 304 and/or within the processing device 302 during execution thereof by the computing device 300, the main memory 304 and the processing device 302 also constituting computer-readable storage media.

The various methods described above may be implemented by a computer program. The computer program may include computer code arranged to instruct a computer to perform the functions of one or more of the various methods described above. The computer program and/or the code for performing such methods may be provided to an apparatus, such as a computer, on one or more computer readable media or, more generally, a computer program product. The computer readable media may be transitory or non-transitory. The one or more computer readable media could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD

In an implementation, the modules, components and other features described herein (for example control unit 310 in relation to Figure 5) can be implemented as discrete components or integrated in the functionality of hardware components such as ASICS, FPGAs, DSPs or similar devices as part of an individualization server.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may be or include a special-purpose processor, such as a field programmable gate array (FPGA) or an ASIC. A hardware component may also include programmable logic or circuitry that is temporarily configured by software to perform certain operations.

Accordingly, the phrase "hardware component" should be understood to encompass a tangible entity that may be physically constructed, permanently configured (e.g., hardwired), or temporarily configured (e.g., programmed) to operate in a certain manner or to perform certain operations described herein.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

## Claims

1. A non-transitory computer readable storage medium for use in detecting and/or monitoring a change in a level of fluid in living tissue, wherein said medium is configured to store instructions that when executed cause a processor to perform:
comparing data representative of one or more parameters extracted from an artificial image placed on a portion of the living tissue with a reference model;
wherein the reference model is predetermined based on one or more parameters extracted from the artificial image before it is placed on the portion of the living tissue and at least one patient-specific parameter.

2. The non-transitory computer-readable storage medium according to claim 1, wherein the instruction configured that when executed to cause the processor to detect and/or to monitor whether the one or more parameters of the artificial pattern exceeds and/or is within a threshold value predetermined by the reference model based on comparison of one or more parameters of the artificial pattern and the reference model; and to generate an alert output when the one or more parameters of the artificial pattern exceeds the predetermined threshold value.

3. The non-transitory computer-readable storage medium according to claim 2, wherein the alert output comprises an indicative that the level of fluid in the living tissue has been changed or it is changing.

4. The non-transitory computer-readable storage medium of any of the preceding claims, wherein the one or more parameters extracted from the artificial image comprises at least one from the group consisting of circumference of the artificial pattern, shape of the artificial image, 2D image of the artificial image, 3D image of the artificial image, volume of the artificial image, color of the artificial pattern, contour of the artificial image, orientation of the artificial image, resolution of the artificial image, stretch of the image.

5. The non-transitory computer-readable storage medium of any of the preceding claims, wherein the at least one patient-specific parameter comprises at least one from the group consisting of soft and connective tissue structure of the portion of the living tissue, skin texture of the portion of the living tissue, muscle tone of the portion of the living tissue, patient's age, patient's weight, patient's activity level, and patient's medical condition.

6. The non-transitory computer-readable storage medium of any of the preceding claims, wherein the non-transitory computer-readable storage medium configured to store instructions that when executed cause a processor to compare plurality of parameters during a first period of time and to compare plurality of parameters during a second period of time; generating an output indicative progression of change in one or more parameters of the artificial image based on comparison with a predetermined threshold value as set by the reference model.

7. A device, comprising:
a transceiver configured to receive data representative of one or more parameters extracted from an artificial image placed on a portion of the living tissue;
a processor operatively coupled to the transceiver;
one or more non-transitory computer readable storage medium according to claims 1-6 operatively connected to the processor.

8. A method for monitoring and/or detecting a change in a level of fluid in living tissue, comprising:
comparing data representative of one or more parameters extracted from an artificial image placed on a portion of the living tissue with a reference model, wherein the reference model is predetermined based on one or more parameters extracted from the artificial image before it is placed on the portion of the living tissue and at least one patient-specific parameter;
detecting and/or monitoring whether the one or more parameters of the artificial pattern exceeds and/or is within a threshold value predetermined by the reference model based on comparison of one or more parameters of the artificial pattern and the reference model; and
generating an alert output when the one or more parameters of the artificial pattern exceeds the predetermined threshold value.

9. The method of claim 8, wherein the alert output comprises an indicative that the level of fluid in the living tissue has been changed or it is changing.

10. The method of claim 8, wherein the one or more parameters extracted from the artificial image comprises at least one from the group consisting of circumference of the artificial pattern, shape of the artificial image, 2D image of the artificial image, 3D image of the artificial image, volume of the artificial image, color of the artificial pattern, contour of the artificial image, orientation of the artificial image, resolution of the artificial image, stretch of the image.

11. The method of claim 8, wherein the at least one patient-specific parameter comprises at least one from the group consisting of soft and connective tissue structure of the portion of the living tissue, skin texture of the portion of the living tissue, muscle tone of the portion of the living tissue, patient's age, patient's weight, patient's activity level, and patient's medical condition.

12. The method of claim 8, further comprises comparing plurality of parameters during a first period of time and to compare plurality of parameters during a second period of time; generating an output indicative progression of change in one or more parameters of the artificial image based on comparison with a predetermined threshold value as set by the reference model.

13. The method of claim 8, wherein detecting a change in the level of fluid in living tissue exceeding the predetermined threshold value set by the reference model is indicative of presence of edema.
